# EUROPÄISCHE PATENTSCHRIFT

(11) **EP 3 233 167 B1**
(45) Veröffentlichungstag und Bekanntmachung des Hinweises auf die Patenterteilung: **23.05.2018**
(21) Anmeldenummer: 15816173.7
(22) Anmeldetag: 18.12.2015
(51) Int. Cl.: A61M 16/04

(54) **TRACHEOSTOMASCHUTZ**
TRACHEOSTOMA PROTECTION
PROTECTION TRACHÉOTOMIQUE

(30) Priorität: 18.12.2014 DE 102014018678
(43) Veröffentlichungstag der Anmeldung: 25.10.2017
(73) Patentinhaber: Andreas Fahl Medizintechnik-Vertrieb GmbH, 51149 Köln (DE)
(72) Erfinder: FAHL, Andreas, 50676 Köln (DE)
(74) Vertreter: Geskes, Christoph
(86) Internationale Anmeldenummer: PCT/EP2015/080678
(87) Internationale Veröffentlichungsnummer: WO 2016/097383

(56) Entgegenhaltungen:
- DE-T2- 69 817 327
- DE-U1- 20 119 493
- DE-U1-202004 010 800
- DE-U1-202013 010 194
- US-A1- 2005 066 816
- US-A1- 2009 227 972

## Beschreibung

Die Erfindung betrifft einen Tracheostomaschutz zur Abdeckung eines Tracheostomas, umfassend ein offenporiges Schaumstoffläppchen.

Vorrichtungen zum Schützen von Tracheostomata sind aus dem Stand der Technik allgemein bekannt. So beschreibt die DE 698 17 327 T2 eine Atemschutzausrüstung für Patienten mit einem Tracheostoma. Diese umfasst einen hoch aufbauenden Feuchte-Wärme-TauscherKörper aus einem offenporigen Schaumstoff, der nahe am und um das Tracheostoma am Hals oder einem Tracheostomawulst klebend befestigt wird. Die distale Seite der Vorrichtung ist mit einer Abdeckplatte versehen, die den Luftstrom ablenkt.

Nachteil an dem aus dem Stand der Technik bekannten Tracheostomaschutz ist, dass dieser schwer handhabbar ist. Insbesondere ist der beschriebene Tracheostomaschutz bei Verwendung einer Stimmprothese unkomfortabel. Um den Sprechvorgang einzuleiten wird das Stoma verschlossen, so dass die Luft beim Ausatmen durch das Sprechventil geleitet wird.

Wird beispielsweise ein Verschließen mittels Zusammendrücken des aus dem Stand der Technik bekannten Tracheostomaschutz erwirkt, wird die Feuchtigkeit, die im hochbauenden Schaumstoff vorhanden ist, ausgedrückt und fließt in die Luftröhre des Patienten oder verunreinigt seine Kleidung. Ebenfalls wird durch die austretende Flüssigkeit der Kleber gelöst. Auch bei der Anbringung des bekannten Tracheostomaschutzes kann es zu Handhabungsschwierigkeiten kommen: So muss der bekannte Tracheostomaschutz an den dem Tracheostoma angrenzenden Teil der Haut befestigt werden. Hierbei werden lediglich kleine Toleranzen geduldet, da ein versetztes Aufkleben bedeutet, dass der Klebeteil des Tracheostomaschutzes das Stoma zumindest teilweise überragt und ein Atmen behindern kann. Die in der DE 698 17 327 T1 beschriebenen Ausgestaltungen verkleinern zudem den effektiven Luftzufuhrdurchmesser, beziehungsweise den Durchmesser des Tracheostomas. Zudem reizt das Klebemittel - wie hautfreundlich dieser auch immer ausgestaltet ist - die Haut, insbesondere die hoch empfindliche Haut des Tracheostomawulstes beziehungsweise das Narbengewebe um das Tracheostoma. Dokumente DE 202004010800 U1 und DE 202013010194 U1 offenbaren auch Tracheostomaschutzeinrichtungen. Aufgabe der Erfindung ist es, die aus dem Stand der Technik bekannten Nachteile zu vermeiden.

Die Aufgabe wird erfindungsgemäß gelöst mittels eines Tracheostomaschutzes nach Anspruch 1 und einer Verwendung eines Tracheostomaschutzes nach Anspruch 11. Weitere vorteilhafte Ausgestaltungen gehen aus den Unteransprüchen, der Beschreibung sowie den Figuren hervor. Einzelne Merkmale der unterschiedlichen Ausgestaltungen können miteinander oder mit weiteren Merkmalen kombiniert werden.

Es wird ein Tracheostomaschutz zur Abdeckung eines Tracheostomas vorgeschlagen, der ein offenporiges Schaumstoffläppchen, einen Klebestreifen, der als doppelseitiges Klebeband ausgestaltet ist, wobei das doppelseitige Klebeband als Klebering ausgestaltet ist, wobei ein äußerer Rand des Kleberings etwa gleich einer Außenkante einer proximalen Oberfläche des Schaumstoffläppchens ist, wobei der Klebestreifen derart ausgestaltet ist, dass das Schaumstoffläppchen beim Ausatmen zumindest teilweise nach distal bewegbar ist, und einen distal auf dem Schaumstoffläppchen angeordneten und dem Tracheostoma zuordbaren luftundurchlässigen Bereich umfasst. Mittels des luftundurchlässigen Bereiches ist das Tracheostoma verdeckbar und verschließbar. Ein Durchmesser des luftundurchlässigen Bereiches ist wesentlich kleiner als der transversale Durchmesser und der longitudinale Durchmesser des Schaumstoffläppchens. Bei Gebrauch des Tracheostomaschutzes ist das Schaumstoffläppchen von einer Berührungsfläche zumindest teilweise nach distal wegbewegbar.

Werden im Rahmen der Beschreibung der Erfindung Richtungsangaben verwendet, so sind diese im Bezug auf den üblichen Gebrauch des Tracheostomaschutzes zu verstehen. Richtungsbezeichnungen werden wie in der Anatomie üblich verwendet. Unter dem Begriff "distal" wird im Sinne der vorliegenden Erfindung in Bezug auf ein Merkmal der erfindungsgemäßen Vorrichtung eine Anordnung bzw. Verwendung desselben fern oder auch abgewandt oder gegenüberliegend eines Tracheostomas oder einer Hautoberfläche eines Benutzers, der insbesondere den erfindungsgemäßen Tracheostomaschutz trägt, verstanden. Unter distal ist vorzugsweise eine Richtung vom Körper des Patienten weg zu verstehen. Unter dem Begriff "proximal" wird im Sinne der vorliegenden Erfindung in Bezug auf ein Merkmal der erfindungsgemäßen Vorrichtung eine Anordnung oder Verwendung desselben nah oder auch zugewandt oder benachbart eines Tracheostomas oder einer Hautoberfläche eines Benutzers, der insbesondere den erfindungsgemäßen Tracheostomaschutz trägt, verstanden. Unter proximal ist vorzugsweise eine Richtung zum Körper des Patienten hin zu verstehen. Unter "transversal" ist vorzugsweise eine Richtung seitlich nach rechts und/oder links von einer Medianebene beziehungsweise einer Medianlinie aus zu verstehen. Unter "longitudinal" ist vorzugsweise eine Richtung längs der Medianebene beziehungsweise einer Medianlinie, insbesondere der Körperlängsachse, nach oben und/oder unten zu verstehen. Unter "superior" ist vorzugsweise eine relative Richtungsangabe längs oder parallel der Körperlängsachse oberhalb eines Bezuges zu verstehen. Unter "inferior" ist vorzugsweise eine relative Richtungsangabe längs oder parallel der Körperlängsachse unterhalb eines Bezuges zu verstehen.

Im Sinne der Erfindung ist die Berührungsfläche, der Teil eines Patienten, insbesondere ein Hautabschnitt, an dem der Tracheostomaschutz beim Einatmen anliegt.

Der Tracheostomaschutz wie vorgeschlagen ist angenehm und diskret zu tragen und vermindert Schabungen an einer Haut des Patienten, die bei aus dem Stand der Technik bekannten Sprechventilen bekannt sind. Nicht zuletzt ist ein solcher Tracheostomaschutz einfach und günstig zu fertigen. Weiterhin vorteilhaft bleibt der Durchmesser des Tracheostoma erhalten, so dass die Atmung auch mit dem vorgeschlagen Tracheostomaschutz nahezu ungestört ist.

Der Tracheostomaschutz wird vorzugsweise über dem Tracheostoma eines Patienten befestigt, bevorzugt so, dass der luftundurchlässige Bereich im Wesentlichen über dem Tracheostoma angeordnet ist, insbesondere nur auf der distalen Seite des Schaumstoffläppchens. Wird im Rahmen der Erfindung der Begriff "etwa" in Bezug auf Werte oder Wertbereiche verwendet, so ist darunter ein Toleranzbereich zu verstehen, den der Fachmann auf diesem Gebiet für üblich erachtet, insbesondere ist ein Toleranzbereich von ±20%, bevorzugt ±10% von den angegebenen Werten vorgesehen. Auch der Begriff "im Wesentlichen" gibt einen Toleranzbereich an, der für den Fachmann unter wirtschaftlichen und technischen Gesichtspunkten zu vertreten ist, sodass das entsprechende Merkmal noch als solches zu erkennen ist.

Unter dem Begriff Klebestreifen sind im Rahmen der Erfindung auch Klebebereiche und/oder Klebepunkte zu verstehen. Erfinderisch ist der zumindest eine Klebestreifen ein doppelseitiges Klebeband. Erfinderisch ist das doppelseitige Klebeband als Klebering ausgestaltet. Im Sinne der Erfindung ist unter einem Ring, beispielsweise einem Klebering, eine Fläche zwischen zwei in einer Ebene liegenden Liniengeometrien, die keine Schnittpunkte aufweisen, beispielsweise ein Kreisring, ein Rechteckring und/oder eine schildförmiger Ring, zu verstehen. Erfinderisch ist ein äußerer Rand des Rings etwa gleich einer Außenkante einer proximalen Oberfläche des Schaumstoffläppchens ausgebildet. Beispielsweise entspricht der äußere Rand der proximalen Oberfläche des Schaumstoffläppchens der Form eines Schildes, das beispielsweise eine U-förmige beziehungsweise halbkreisförmig abgerundete Spitze aufweist. Beispielsweise kann dann der Klebestreifen, der als Klebeband ausgestaltet ist, ein Ring, der ebenfalls die Form eines Schildes aufweist, sein. Der äußere Rand des Rings gleicht etwa der Außenkante der proximalen Oberfläche des Schaumstoffläppchens beziehungsweise grenzt an diese Außenkante an.

Gemäß einer Ausgestaltung weist das Schaumstoffläppchen in einer Aufsicht von distal eine im Wesentlichen rechteckige Ausgestaltung auf, vorzugsweise mit abgerundeten Ecken. In einer weiteren Ausgestaltung weißt das Schaumstoffläppchen eine gerundete Form auf, beispielsweise eine Tropfenform, eine elliptische Form, eine kreisrunde Form. In einer weiteren Ausführungsform weist das Schaumstoffläppchen in der Aufsicht eine beliebige Form auf. Gemäß einer Ausführungsform ist vorgesehen, dass der Klebestreifen ein auf dem Schaumstoff aufgebrachter Kleber ist. Erfindungsgemäß ist der Klebestreifen als ein doppelseitiges Klebeband ausgestaltet das auf dem Schaumstoffläppchen aufgebracht ist. Vorzugsweise ist der Klebestreifen auf der proximalen Seite des Schaumstoffläppchens angeordnet. In einer weiteren Ausgestaltung ist vorgesehen, dass das Klebeband ein Ringpflaster umfasst. Vorteilhafterweise ist durch den Klebestreifen gewährleistet, dass trotz der Wegbewegung des Schaumstoffläppchens beim Ausatmen der Tracheostomaschutz am Hals des Patienten befestigt bleibt. Erfindungsgemäß ist bei den unterschiedlichen Ausführungsformen des Klebestreifens gewährleistet, dass das Schaumstoffläppchen insbesondere beim Ausatmen zumindest teilweise durch den Luftstrom von dem Tracheostoma nach distal wegbewegbar ist.

Vorteil des vorgeschlagenen Tracheostomaschutzes ist, dass durch den luftundurchlässigen Bereich eine Sprechfunktion einfach verwirklicht werden kann, indem ein leichter Fingerdruck auf eben diesen Bereich ausgeübt wird und auf diese Weise das Tracheostoma verschlossen wird. Weiterhin vorteilhaft wird die Atmung im Gegensatz zu den aus dem Stand der Technik bekannten Sprechventilen nicht behindert. Vielmehr kann insbesondere ein Ausatmen leichter erfolgen, da das Schaumstoffläppchen zumindest teilweise von einer Berührungsebene nach distal bewegbar ist. Der Luftstrom kann in einer Ausgestaltung auch an dem Schaumstoffläppchen vorbeiströmen. Vorteilhaft an den beschriebenen Ausgestaltungen ist, dass der Luftstrom insbesondere beim Ausatmen in einer Ausführungsform zu einem Teil an dem Schaumstoffläppchen vorbei strömt. Hierdurch ist ein geringer Widerstand beim Ausatmen gegeben, der insbesondere bei sportlichen Aktivitäten als unangenehm empfunden wird.

In einer weiteren Ausgestaltung ist vorgesehen, dass bei Gebrauch des Tracheostomaschutzes ein Luftstrom während des Einatmens im Wesentlichen insbesondere von distal nach proximal durch das Schaumstoffläppchen strömt. Vorzugsweise legt sich das Schaumstoffläppchen an den Hals des Patienten beziehungsweise die Berührungsebene derart an, dass beim Einatmen der Luftstrom nur durch das Schaumstoffläppchen hindurch erfolgt. Besonders vorteilhaft ist dies, da gerade beim Einatmen das Tracheostoma vor Fremdkörpern geschützt werden muss. Weiterhin vorteilhaft ist, dass der Luftstrom durch den Tracheostomaschutz vorgewärmt und angefeuchtet wird, bevor dieser in die Trachea, die Luftröhre, gelangt.

In einer weiteren Ausführungsform ist vorgesehen, dass bei Gebrauch des Tracheostomaschutzes ein Luftstrom während des Ausatmens im Wesentlichen von proximal nach distal strömt. Insbesondere ist dies der Fall, wenn das Schaumstoffläppchen an zumindest zwei Seiten an der Berührungsebene gehalten ist, beispielsweise durch den Klebestreifen und/oder durch ein Kleidungsstück. Sind zwei oder mehr Klebestreifen vorgesehen, so können diese longitudinal und/oder transversal zu dem luftundurchlässigen Bereich angeordnet sein. Das Halten des Schaumstoffläppchens an zumindest zwei Seiten oder durch ein Ringpflaster, das heißt einen ringförmigen Klebestreifen, beeinflusst in einer Ausführungsform die Wegbewegung nach distal von der Berührungsebene nicht. Vielmehr wird auf diese Weise ein Aufblähen des Tracheostomaschutzes beziehungsweise des Schaumstoffläppchens bewirkt, was ebenfalls in einer Ausgestaltung unter der teilweisen Wegbewegung des Schaumstoffläppchens subsumiert wird. Wird das Schaumstoffläppchen teilweise beim Ausatmen wegbewegt, erfolgt dennoch ein Teilstrom der Luft in das Schaumstoffläppchen, so dass dieses angefeuchtet und angewärmt wird.

Vorteilhaft an den beschriebenen Ausgestaltungen ist, dass der Luftstrom insbesondere beim Ausatmen in einer Ausführungsform im Wesentlichen von proximal nach distal durch das Schaumstoffläppchen strömt. Ein laterales oder longitudinales Ausströmen der Luft, insbesondere parallel zur Haut, aus dem Tracheostomaschutz beim Ausatmen wird oft als unangenehm empfunden, da insbesondere bei kalter Witterung die Haut um das Tracheostoma beziehungsweise um den Tracheostomaschutz auskühlt. Ein Ausströmen der Luft im Wesentlichen von proximal nach distal hält die feuchte Ausatemluft von der umgebenen Haut fern. Vorzugsweise ist der luftunduchlässige Bereich derart angeordnet, dass ein Umströmen dessen durch ausgeatmete Luft gewährleistet ist. Vorteilhafterweise wird die Luftführung mittels der lateralen und/oder longitudinalen Erstreckung des Tracheostomaschutzes und/oder der Dicke des Schaumstoffläppchens erzielt. Weiterhin vorteilhaft wird in einer Ausgestaltung mit einem im Wesentlichen ringförmigen Klebestreifen gewährleistet, dass durch das gezielte Aufblähen des Tracheostomaschutzes die Ausatemluft in Verbindung mit dem Schaumstoffläppchen, das eine das Tracheostoma umgebene Haut bedeckt, die Haut um das Stoma warm hält.

Bevorzugt strömt bei Gebrauch des Tracheostomaschutzes zumindest ein Teil eines Luftstroms während des Ausatmens an dem Schaumstoffläppchen vorbei.

In einer weiteren Ausgestaltung ist vorgesehen, dass das Schaumstoffläppchen einen transversalen und/oder longitudinalen Durchmesser aufweist, der zumindest doppelt so groß wie ein Durchmesser des luftundurchlässigen Bereiches ist. Vorteil an diesem Merkmal ist, dass der Tracheostomaschutz bequemer zu tragen ist als Vorrichtungen aus dem Stand der Technik. Insbesondere Schabungen und Hautirritationen durch die an dem Hals anliegenden Materialien werden durch die große Auflagefläche vermindert. Weiterhin vorteilhaft ist, dass der Tracheostomaschutz einfach zu handhaben ist. So muss dieser nicht exakt über dem Tracheostoma platziert werden, sondern erlaubt größere Toleranzen als bei aus dem Stand der Technik bekannten Sprechventilen, um zumindest eine Feuchte-Wärme-Tauscherfunktion zu gewährleisten. Ein weiterer Vorteil ist, dass der Klebestreifen weit genug vom Tracheostoma platziert werden kann, so dass das empfindliche Gewebe um das Tracheostoma, beziehungsweise der Tracheostomawulst nicht durch den Kleber oder das tägliche Wechseln des Tracheostomaschutzes gereizt wird.

Weiterhin vorteilhaft an dieser Ausgestaltung ist, dass die Feuchtigkeit weit vom Tracheostoma weg transportierbar ist, vorzugsweise aufgrund des Kapillareffektes des Schaumstoffs. Bei aus dem Stand der Technik bekannten Sprechventilen bleibt die Feuchtigkeit in der Nähe des Tracheostomas, sodass bei einem Druck auf den Schaumstoff des Sprechventils diese ausgedrückt wird und Flüssigkeit in die Trachea oder die Kleidung des Patienten fließt.

In einer weiteren Ausführungsform ist vorgesehen, dass das Schaumstoffläppchen eine Dicke von etwa 1 mm bis etwa 8 mm, bevorzugt etwa 1 mm bis etwa 6 mm, weiter bevorzugt etwa 2 mm bis etwa 5 mm aufweist. Vorteilhafterweise wird dadurch eine geringe Aufbauhöhe erreicht. Der Tracheostomaschutz fällt wenig auf und ist angenehm zu tragen, da beispielsweise Kleidungsstücke kaum auf diesen einwirken. Weiterhin fasst der Tracheostomaschutz je Quadratzentimeter Oberfläche weniger Flüssigkeit, als aus dem Stand der Technik bekannte Sprechventile. Auf diese Weise wird ebenfalls ein Ausdrücken von Flüssigkeit aus dem Schaumstoffläppchen beim Einleiten der Sprechfunktion vermindert oder vermieden. Weiterhin vorteilhaft ist, dass ein Anpressdruck zum Verschließen des Tracheostomas wesentlich geringer ausgeübt werden muss als bei den aus dem Stand der Technik bekannten Sprechventilen, so dass ein unangenehmer Druck auf den Tracheostomawulst vermindert werden kann und dennoch die Funktionalität erhalten bleibt.

In einer weiteren Ausgestaltung ist vorgesehen, dass der luftundurchlässige Bereich einen Knopf, eine Platte und/oder eine Verhautung des Läppchens umfasst. Vorteilhafterweise erleichtert der luftundurchlässige Bereich ein Verschließen des Tracheostomas und gibt dem Patienten Rückmeldung darüber, dass er an der richtigen Stelle drückt. Dies kann durch eine Oberflächengestaltung zur Erzielung einer besonderen Optik und/oder Haptik oder durch eine relativ zum Schaumstoffläppchen größere Rigidität erzielt werden. Insbesondere weist der Knopf oder die Platte gute Rückmeldeeigenschaften auf. Umfasst der undurchlässige Bereich einen Knopf oder eine Platte aus einem relativ zu einem Schaumstoffmaterial des Schaumstoffläppchens rigiden Material, erleichtert dies ein Verdichten des Schaumstoffmaterials für den Benutzer beim Einleiten der Sprechfunktion.

In einer Ausführungsform ist vorgesehen, dass der Knopf oder die Platte aufgeklebt, verschweißt und/oder angenäht ist. Weiterhin sieht eine Ausgestaltung vor, dass der Knopf oder die Platte angeschweißt ist. Gemäß einer Variante ist der Schaumstoff an den Knopf oder die Platte angespritzt beziehungsweise angeschäumt. In einer Ausgestaltung, bei der eine Verhautung des Schaumstoffläppchens vorgesehen ist, wird die Verhautung mittels Aufschmelzen und Erkalten lassen eines Oberflächenteilbereiches des Schaumstoffläppchens erzeugt. In einer weiteren Ausgestaltung ist vorgesehen, dass die Verhautung eine aufgeklebte oder eine aufgeschweißte Folie umfasst.

In einer weiteren Ausführungsform ist vorgesehen, dass der luftundurchlässige Bereich im Gebrauch zur Vermittlung einer Sprechfunktion nach proximal bewegbar ist. Insbesondere ist vorgesehen, dass proximal des luftundurchlässigen Bereichs ein Schaumstoffmaterial vorgesehen ist, der zusammendrückbar beziehungsweise verdichtbar ist. Wenn der luftundurchlässige Bereich nach proximal bewegt wird, verdichtet sich das Schaumstoffmaterial des Schaumstoffläppchens im Bereich des Tracheostomas. In einer weiteren Ausgestaltung ist vorgesehen, dass mittels des luftundurchlässigen Bereiches das Tracheostoma verschließbar ist. Insbesondere ist der luftundurchlässige Bereich so nahe an das Tracheostoma bewegbar, dass keine Luft aus dem Tracheostoma entweichen kann. Da das Schaumstoffläppchen sehr dünn ausgeführt ist, könnte unter Umständen das Verdichten des Schaumstoffmaterials nicht ausreichen, um eine ausreichende Luftundurchlässigkeit zu gewährleisten. Durch den luftundurchlässigen Bereich wird jedoch sichergestellt, dass nur sehr wenig bis keine Luft bei Einleitung des Sprechvorganges bei Betätigung des Tracheostomaschutzes beispielsweise durch Fingerdruck aus dem Tracheostoma entweichen kann. Gleichzeitig wird eine komfortable Atmung aufgrund der distalen Lage des luftundurchlässigen Bereiches gewährleistet.

In einer weiteren Ausführungsform ist vorgesehen, dass der Klebestreifen derart an dem Schaumstoffläppchen angeordnet ist, dass dieser im Wesentlichen superior des Tracheostomas befestigbar ist. Der Klebestreifen ist insbesondere ein gerader oder gekrümmter Streifen. In einer Ausgestaltung ist vorgesehen, dass eine Anzahl von Klebestreifen, Klebebereichen und/oder Klebepunkten auf dem Schaumstoffläppchen vorgesehen ist. Weiterhin kann sich der Klebestreifen auch zumindest teilweise um das Tracheostoma beziehungsweise um den luftundurchlässigen Bereich erstrecken. In einer weiteren Ausgestaltung ist vorgesehen, dass sich der Klebestreifen im Wesentlichen vollständig um das Tracheostoma beziehungsweise um den luftundurchlässigen Bereich erstreckt. In einer weiteren Ausgestaltung ist vorgesehen, dass der Klebestreifen im Bereich einer longitudinalen und/oder transversalen Kante des Schaumstoffläppchens angeordnet ist. In einer weiteren Ausführungsform ist vorgesehen, dass der Klebestreifen im Wesentlichen angrenzend an eine laterale und/oder longitudinale Kante des Schaumstoffläppchens angeordnet ist. In einer weiteren Ausführungsform ist vorgesehen, dass der Klebestreifen, vorzugsweise auf der proximalen Seite, auf dem Schaumstoffläppchen, vorzugsweise nahe an einer Kante des Schaumstoffläppchens, bevorzugt im Wesentlichen abschließend mit einer Kante des Schaumstoffläppchens, weiter bevorzugt bis zu einer lateralen und/oder longitudinalen Kante des Schaumstoffläppchens, angeordnet ist. In einer weiteren Ausgestaltung ist der Klebestreifen ringförmig ausgestaltet. In einer bevorzugten Ausgestaltung ist der Klebestreifen als ein Ring, der im Wesentlichen eine Außenform aufweist, die der Außenform des Schaumstoffläppchens entspricht, ausgestaltet. In einer weiteren Ausgestaltung ist vorgesehen, dass der Klebestreifen superior des Tracheostomas beziehungsweise des luftundurchlässigen Bereiches zumindest teilweise breiter ausgestaltet ist als der Rest des Klebestreifens. In einer weiteren Ausführungsform ist vorgesehen, dass der Klebestreifen die Form eines Ringsegments umfasst. In einer weiteren Ausgestaltung ist vorgesehen, dass ein Klebestreifen zusätzlich oder alternativ zu weiteren Klebestreifen inferior des Tracheostomas befestigbar ist.

Schließlich wird eine Verwendung eines wie oben beschriebenen Tracheostomaschutzes zum selektiven luftdichten Verschließen eines Tracheostomas vorgeschlagen. Der Tracheostomaschutz kann auch zur Anfeuchtung und/oder Aufwärmung der eingeatmeten Luft verwendet werden. Ebenfalls ist gemäß einer Ausgestaltung vorgesehen, dass der Tracheostomaschutz zur Abhaltung von Fremdkörpern vorgesehen ist, die in die Trachea eindringen könnten.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen:
- Fig. 1: zeigt einen Tracheostomaschutz in einer Aufsicht von distal;
- Fig. 2: zeigt eine weitere Ausgestaltung eines Tracheostomaschutzes in der gleichen Ansicht;
- Fig. 3: zeigt eine Seitenansicht eines Tracheostomaschutzes;
- Fig. 4: zeigt schematisch die Funktionsweise des Tracheostomaschutzes;
- Fig. 5: zeigt schematisch die Funktion des Tracheostomaschutzes beim Einatmen; und
- Fig. 6: zeigt schematisch die Funktionsweise des Tracheostomaschutzes bei Einleitung eines Sprechvorgangs.

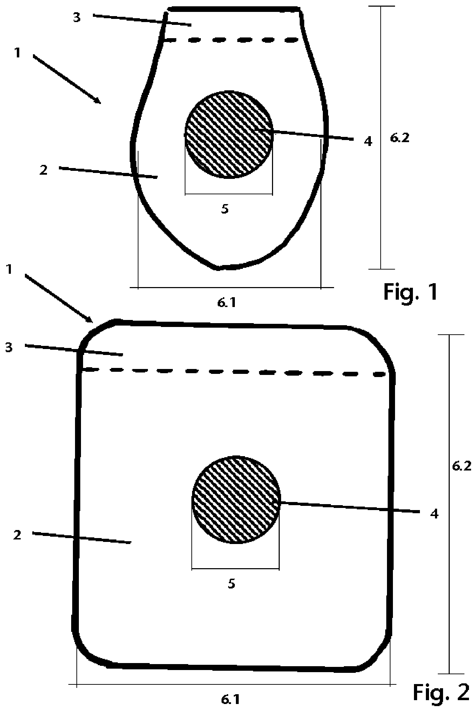

Fig. 1 zeigt einen Tracheostomaschutz 1 in einer Aufsicht von distal. Der Tracheostomaschutz umfasst ein Schaumstoffläppchen 2 sowie einen in der Fig. 1 angedeuteten Klebestreifen 3, der auf der proximalen Seite des Schaumstoffläppchens 2 angeordnet ist. Weiterhin weist der Tracheostomaschutz 1 einen luftundurchlässigen Bereich 4 auf, der vorzugsweise als Knopf ausgestaltet ist. Der Durchmesser 5 des Knopfes ist in der gezeigten Ausführungsform etwa halb so groß wie der transversale Durchmesser 6.1 des Schaumstoffläppchens 2. Auch der longitudinale Durchmesser 6.2 des Schaumstoffläppchens 2 ist doppelt so groß beziehungsweise mehr als doppelt so groß wie der Durchmesser 5 des Knopfes 4.

Fig. 2 zeigt eine weitere Ausgestaltung des Tracheostomaschutzes 1, welcher ebenfalls ein Schaumstoffläppchen 2, einen Klebestreifen 3 auf der proximalen Seite des Schaumstoffläppchens 2 und einen luftundurchlässigen Bereich 4 auf der distalen Seite des Schaumstoffläppchens 2 aufweist. Erfinderisch ist der Durchmesser 5 des luftundurchlässigen Bereiches 4 wesentlich kleiner, insbesondere etwa ein Drittel kleiner, als der transversale Durchmesser 6.1 und der longitudinale Durchmesser 6.2.

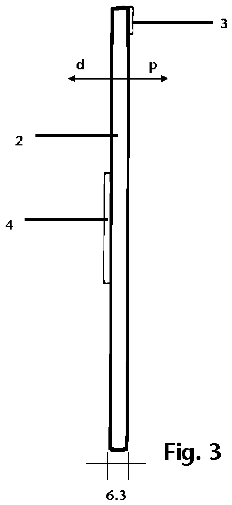

Fig. 3 zeigt einen Tracheostomaschutz 1 in einer Seitenansicht. In der Seitenansicht ist deutlich zu erkennen, dass auf der distalen Seite d der Knopf 4 angeordnet, insbesondere aufgeklebt ist. Auf der proximalen Seite p ist ein Klebestreifen 3, insbesondere ausgestaltet als doppelseitiges Klebeband aufgebracht. Weiterhin ist zu erkennen, dass das Schaumstoffläppchen 2 eine relativ geringe Dicke 6.3 von etwa 2 mm bis etwa 5 mm aufweist.

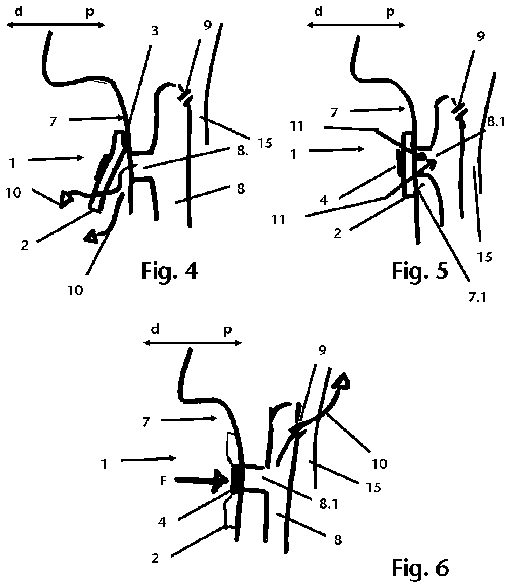

Fig. 4 zeigt eine vereinfachte schematische Darstellung der Verwendung des Tracheostomaschutzes 1. Der Tracheostomaschutz 1 wird mittels des Klebestreifens 3 an einen Hals 7 eines Benutzers beziehungsweise Patienten angeordnet. Beim Ausatmen strömt ein Luftstrom 10 aus der Trachea 8 durch das Tracheostoma 8.1. Der Tracheostomaschutz 1 wird dadurch von dem Hals 7 abgehoben, so dass der Luftstrom 10 zumindest teilweise an dem Tracheostomaschutz 1 vorbeiströmen kann. Ein Teil des Luftstromes 10 durchströmt weiterhin das Schaumstoffläppchen 2 und wärmt und feuchtet dieses an.

Fig. 5 zeigt die gleiche Situation wie Fig. 4, wobei der Benutzer einatmet. Der Luftstrom 11 beim Einatmen durchströmt das Schaumstoffläppchen 2, wobei sich das Schaumstoffläppchen 2 dabei an den Hals 7 des Patienten anlegt und insbesondere an eine Berührungsebene 7.1 des Halses anpasst. Selbst wenn der luftundurchlässige Bereich 4, der als Knopf in dieser Ausgestaltung ausgeführt ist, den Bereich des Tracheostomas 8.1 überdeckt, ist das Schaumstoffläppchen 2 beziehungsweise dessen Dicke derart bemessen, dass der Luftstrom 11 leicht beziehungsweise einfach an dem luftundurchlässigen Bereich 4 vorbeiströmen kann. Eine Abdichtung wird also nur erzeugt, wenn der Benutzer auf den luftundurchlässigen Bereich 4 drückt.

Fig. 6 zeigt den Tracheostomaschutz 1 am Hals 7 des Patienten, wobei eine Kraft F, beispielsweise durch einen Finger ausgeübt, auf den luftundurchlässigen Bereich 4 wirkt. Durch die Ausübung der Kraft wird das Schaumstoffläppchen 2 im Bereich des Tracheostomas 8.1 komprimiert, so dass die ausgeatmete Luft 10 nicht durch das Tracheostoma 8.1 entweichen kann. Stattdessen strömt der Luftstrom 10 durch eine Stimmprothese 9, die die Trachea 8 mit einer Speiseröhre 15 verbindet. Auf diese Weise wird dem Benutzer beziehungsweise dem Patienten ermöglicht zu sprechen.

## Patentansprüche

1. Tracheostomaschutz (1) zur Abdeckung eines Tracheostomas (8), umfassend ein offenporiges Schaumstoffläppchen (2), einen Klebestreifen (3), der als doppelseitiges Klebeband ausgestaltet ist, wobei das doppelseitige Klebeband als Klebering ausgestaltet ist, wobei ein äußerer Rand des Kleberings etwa gleich einer Außenkante einer proximalen Oberfläche des Schaumstoffläppchens (2) ist, wobei der Klebestreifen (3) derart ausgestaltet ist, dass das Schaumstoffläppchen (2) beim Ausatmen zumindest teilweise durch einen Luftstrom von dem Tracheostoma nach distal bewegbar ist, und einen distal auf dem Schaumstoffläppchen (2) angeordneten und dem Tracheostoma (8) zuordbaren luftundurchlässigen Bereich (4), wobei mittels des luftundurchlässigen Bereiches (4) das Tracheostoma (8) verdeckbar und verschließbar ist und wobei ein Durchmesser (5) des luftundurchlässigen Bereiches (4) wesentlich kleiner als der transversale Durchmesser (6.1) und der longitudinale Durchmesser (6.2) des Schaumstoffläppchens (2) ist, und wobei bei Gebrauch des Tracheostomaschutzes (1) das Schaumstoffläppchen (2) von einer Berührungsfläche zumindest teilweise nach distal wegbewegbar ist.

2. Tracheostomaschutz (1) nach Anspruch 1, **dadurch gekennzeichnet, dass** bei Gebrauch des Tracheostomaschutzes (1) ein Luftstrom (11) während des Einatmens im Wesentlichen durch das Schaumstoffläppchen (2) strömt.

3. Tracheostomaschutz (1) nach einem oder mehren der vorhergehenden Ansprüche, **dadurch gekennzeichnet, dass** bei Gebrauch des Tracheostomaschutzes (1) ein Luftstrom (11) während des Ausatmens zumindest teilweise am Schaumstoffläppchen (2) vorbeiströmt.

4. Tracheostomaschutz (1) nach einem oder mehren der vorhergehenden Ansprüche, **dadurch gekennzeichnet, dass** das Schaumstoffläppchen (2) einen transversalen und/oder longitudinalen Durchmesser (6) aufweist, der zumindest doppelt so groß ist wie ein Durchmesser (5) des luftundurchlässigen Bereiches (4) ist.

5. Tracheostomaschutz (1) nach einem oder mehreren der vorhergehenden Ansprüche, **dadurch gekennzeichnet, dass** das Schaumstoffläppchen (2) eine Dicke von etwa 1 mm bis etwa 6 mm aufweist.

6. Tracheostomaschutz (1) nach einem oder mehreren der vorhergehenden Ansprüche, **dadurch gekennzeichnet, dass** der luftundurchlässige Bereich (4) einen Knopf, eine Platte und/oder eine Verhautung des Läppchens (2) umfasst.

7. Tracheostomaschutz (1) nach Anspruch 6, **dadurch gekennzeichnet, dass** der Knopf angeklebt, verschweißt und/oder angenäht ist.

8. Tracheostomaschutz (1) nach einem oder mehreren der vorhergehenden Ansprüche, **dadurch gekennzeichnet, dass** der luftundurchlässige Bereich (4) bei Gebrauch zur Vermittlung einer Sprechfunktion nach proximal bewegbar ist.

9. Tracheostomaschutz (1) nach einem oder mehreren der vorhergehenden Ansprüche, **dadurch gekennzeichnet, dass** der Klebestreifen (3) derart an dem Schaumstoffläppchen (2) angeordnet ist, dass dieser im Wesentlichen superior des Tracheostomas (8) befestigbar ist.

10. Verwendung eines Tracheostomaschutzes (1) nach einem oder mehreren der Ansprüche 1 bis 9 zum selektiven luftdichten Verschließen eines Tracheostomas (8).

## Claims

1. Tracheostoma protection device (1) for covering a tracheostoma (8), comprising an open-cell foam patch (2), an adhesive strip (3) designed as a double-sided adhesive tape, wherein the double-sided adhesive tape is designed as an adhesive ring, wherein an outer edge of the adhesive ring is approximately equal to an outer edge of a proximal surface of the foam patch (2), wherein the adhesive strip (3) is designed in a way that through an airflow upon exhalation, the foam patch (2) can be moved distally, at least partially, away from the tracheostoma, and an air-impermeable region (4) disposed distally on the foam patch (2) and allocated to the tracheostoma (8), wherein the tracheostoma (8) can be covered and closed by the air-impermeable region (4) and wherein a diameter (5) of the air-impermeable region (4) is substantially smaller than the transverse diameter (6.1) and the longitudinal diameter (6.2) of the foam patch (2), and wherein the foam patch (2) can be moved distally, at least partially, away from a contact surface during use of the tracheostoma protection device (1).

2. Tracheostoma protection device (1) according to claim 1, **characterized in that** an airflow (11) flows substantially through the foam patch (2) during inhalation when the tracheostoma protection device (1) is in use.

3. Tracheostoma protection device (1) according to one or more of the preceding claims, **characterized in that** an airflow flows at least in part past the foam patch (2) during exhalation when the tracheostoma protection device (1) is in use.

4. Tracheostoma protection device (1) according to one or more of the preceding claims, **characterized in that** the foam patch (2) has a transverse and/or longitudinal diameter (6) that is at least twice as large as a diameter (5) of the air-impermeable region (4).

5. Tracheostoma protection device (1) according to one or more of the preceding claims, **characterized in that** the foam patch (2) has a thickness of approximately 1 mm to approx. 6 mm.

6. Tracheostoma protection device (1) according to one or more of the preceding claims, **characterized in that** the air-impermeable region (4) comprises a button, a plate and/or a skin of the foam patch (2).

7. Tracheostoma protection device (1) according to claim 6, **characterized in that** the button is glued, welded and/or sewn on.

8. Tracheostoma protection device (1) according to one or more of the preceding claims, **characterized in that** the air-impermeable region (4) can be moved proximally during use, in order to initiate a speech function.

9. Tracheostoma protection device (1) according to one or more of the preceding claims, **characterized in that** the adhesive strip (3) is disposed on the foam patch (2) such that it is attachable substantially superior to the tracheostoma (8).

10. Use of the tracheostoma protection device (1) according to one or more of the claims 1 to 9, for a selective airtight sealing of a tracheostoma (8).

## Revendications

1. Protection trachéotomique (1) pour recouvrir un trachéostome (8), comprenant une pièce en mousse à pores ouverts (2), une bande adhésive (3) qui est constituée comme un ruban adhésif double face, le ruban adhésif double face étant constitué comme un anneau adhésif, un bord extérieur de l'anneau adhésif étant sensiblement équivalent à une bordure extérieure d'une surface proximale de la pièce en mousse (2), la bande adhésive (3) étant constituée de sorte que la pièce en mousse (2) peut être éloignée au moins partiellement par un flux d'air du trachéostome lors de l'expiration, et une zone imperméable à l'air (4) disposée du côté distal sur la pièce en mousse (2) et pouvant être associé au trachéostome (8), le trachéostome (8) pouvant être recouvert et obturé par la zone imperméable à l'air (4) et un diamètre (5) de la zone imperméable à l'air (4) état sensiblement inférieur au diamètre transversal (6.1) et au diamètre longitudinal (6.2) de la pièce en mousse (2), et la pièce en mousse (2) pouvant être éloignée au moins partiellement d'une surface de contact lors de l'utilisation de la protection trachéotomique (1).

2. Protection trachéotomique (1) selon la revendication 1, **caractérisée en ce que**, lors de l'utilisation de la protection trachéotomique (1), un flux d'air (11) traverse sensiblement la pièce en mousse (2) pendant l'inspiration.

3. Protection trachéotomique (1) selon une ou plusieurs des revendications précédentes, **caractérisée en ce que**, lors de l'utilisation de la protection trachéotomique (1), un flux d'air (11) s'écoule au moins partiellement contre la pièce en mousse (2) pendant l'expiration.

4. Protection trachéotomique (1) selon une ou plusieurs des revendications précédentes, **caractérisée en ce que** la pièce en mousse (2) présente un diamètre transversal et/ou longitudinal (6) qui est au moins deux fois plus grand qu'un diamètre (5) de la zone imperméable à l'air (4).

5. Protection trachéotomique (1) selon une ou plusieurs des revendications précédentes, **caractérisée en ce que** la pièce en mousse (2) présente une épaisseur d'environ 1 mm à environ 6 mm.

6. Protection trachéotomique (1) selon une ou plusieurs des revendications précédentes, **caractérisée en ce que** la zone imperméable à l'air (4) comprend un bouton, une plaque et/ou un revêtement de la pièce (2).

7. Protection trachéotomique (1) selon la revendication 6, **caractérisée en ce que** le bouton est collé, soudé et/ou cousu.

8. Protection trachéotomique (1) selon une ou plusieurs des revendications précédentes, **caractérisée en ce que**, lors de l'utilisation, la zone imperméable à l'air (4) peut se rapprocher pour offrir une fonction vocale.

9. Protection trachéotomique (1) selon une ou plusieurs des revendications précédentes, **caractérisée en ce que** la bande adhésive (3) est disposée sur la pièce en mousse (2) de sorte que celle-ci peut être fixée sensiblement au-dessus du trachéostome (8).

10. Utilisation d'une protection trachéotomique (1) selon une ou plusieurs des revendications 1 à 9 pour obturer un trachéostome (8) de manière étanche à l'air de façon sélective.
